# EUROPEAN PATENT SPECIFICATION

(11) **EP 1 727 478 B1**
(45) Date of publication and mention of the grant of the patent: **14.12.2011**
(21) Application number: 05708953.4
(22) Date of filing: 07.03.2005
(51) Int. Cl.: A61B 17/12

(54) **APPARATUS AND METHOD FOR THERAPEUTIC EMBOLIZATION**
GERÄT UND VERFAHREN FÜR DIE THERAPEUTISCHE EMBOLISATION
DISPOSITIF ET PROCEDE D'EMBOLISATION THERAPEUTIQUE

(30) Priority: 10.03.2004 EP 04100968
(43) Date of publication of application: 06.12.2006
(73) Proprietor: Philips Intellectual Property & Standards GmbH, 20099 Hamburg (DE); Koninklijke Philips Electronics N.V., 5621 BA Eindhoven (NL)
(72) Inventor: BORGERT, Joern, Weisshausstr. 2, 52066 Aachen (DE); BABIC, Drazenko Mihovil, Weisshausstr. 2, 52066 Aachen (DE)
(86) International application number: PCT/IB2005/050823
(87) International publication number: WO 2005/087114

(56) References cited:
- US-A- 5 042 486
- US-A- 5 443 454
- US-B1- 6 364 823
- US-B1- 6 522 909

## Description

The invention relates to an apparatus, and a method for the therapeutic embolization of aneurysms using a catheter.

In the therapeutic embolization of aneurysms, cavities caused by disorders in the vascular system are eliminated by injecting a suitable plugging material, so that negative effects of the aneurysm on the blood flow are suppressed. A number of variants of therapeutic embolization are known from the literature. For example, US 6 024 754 describes the combination of a coil firstly inserted into the aneurysm with a curable polymer which is subsequently introduced. X-ray-opaque markers are fitted to the tip of the associated catheter, it being possible for the position of said markers to be ascertained on an X-ray image. Furthermore, US 6 024 754 indicates the known requirement, during injection of the plugging material into the aneurysm, to take care that the catheter tip does not emerge from the aneurysm and discharge plugging material into the blood stream or block the blood vessel. However, no further details are given with regard to how this aim is to be achieved.

US 5,443,454 describes a catheter for embolectomy having an opening of an insertion hole , which opening communicates with an proximal end of the catheter and permits insertion of a guide wire or an endoscope.

US 5,042,486 describes a catheter being locatable with a non-ionizing field and method for locating the same.

Against this background, it is an object of the present invention to provide means for safely carrying out a therapeutic embolization using a catheter.

This object is achieved by an apparatus, and by a method of operating the apparatus according to the independent claims.

Advantageous embodiments are given in the dependent claims.

There is provided a catheter for the therapeutic embolization of aneurysms by injection of a filling material into the aneurysm by way of an active locator for the determination of the spatial position and/or orientation of the catheter.

According to an exemplary embodiment the apparatus according to the invention is used for the therapeutic embolization of aneurysms and contains the following components:
- A catheter for injecting a filling material into an aneurysm.
- A locating device and at least one associated active locator which is fitted on the catheter (preferably at the tip thereof), it being possible for the spatial position and/or orientation of the locator to be determined by the locating device. The term "active locator"is to be understood here to mean a device whose spatial position/orientation can be determined not only passively, that is to say using imaging methods (such as the X-ray imaging of an X-ray-opaque marker for example), but also, as an alternative or in addition, in some other way. Typically, active locators transmit or receive appropriate signals which serve for position determination purposes.
- A pump device for controllably supplying filling material to the catheter.
- A monitoring unit connected to the locating device and the pump device, for example a microcomputer with appropriate software, wherein the monitoring unit is designed to detect emergence of the catheter from the aneurysm during the injection of plugging material into the aneurysm, and thereupon to immediately stop the supply of plugging material to the catheter. The monitoring unit is able to detect emergence of the catheter from the aneurysm by virtue of its connection to the locating device, which locating device continuously measures the position and/or orientation of the active locator on the catheter. Stopping of the supply of plugging material is achieved by correspondingly controlling the pump device. The monitoring unit, the locating device and the pump device may be -separates physical units; however, they may also be completely or partially embodied in a single unit, for example a microcomputer.

The described apparatus has the advantage that it allows particularly safe embolization of aneurysms. This is achieved in that the catheter tip is observed and any emergence thereof from the aneurysm is detected and thereupon the supply of plugging material is automatically stopped. Therefore, no plugging material, or at most a minimal amount of plugging material, can be discharged into the bloodstream. The apparatus thus ensures safety even if, despite the greatest of care being taken, the catheter tip slides out of the aneurysm. Since an active locator is used, the catheter can in this case be monitored without continuous X-ray-fluoroscopic observation, and this entails a corresponding reduction in the amount of radiation to which the patient and the staff are exposed. A further advantage of the apparatus is that the locator present at the catheter tip can also be used for further functions, for example for a local improvement in the imaging of a region of interest by temporal integration of image information, and also for navigating the catheter in the vascular system of a patient.

According to one further embodiment of the apparatus, the monitoring unit contains a memory with a road map stored therein and also means of recording the measured position of the locator using this road map. Two-dimensional or three-dimensional road maps show the course of a patient's vessels and are often generated prior to or at the start of an intervention by means of an X-ray angiograph, in order to be able to carry out the subsequent navigation in the vascular system without continuously exposing the patient to X-ray radiation and contrast agent. During the intervention, the position of the catheter is measured using other means such as, for example, the active locator and recorded using the road map, that is to say is assigned to the geometrically and/or anatomically corresponding point of the road map. Since the road map contains in particular the position of the aneurysm, it can be checked whether, at the start of embolization, the catheter is at the desired location relative to the course of the vessels-i. e. in the aneurysm.

The apparatus may furthermore optionally comprise an imaging device such as in particular an X-ray device. It is thereby possible if necessary to generate current images and thus to check the position of the catheter relative to the course of the vessels. In particular, it is possible in this way to check the correct positioning of the catheter at the start of embolization. Furthermore, the catheter may preferably carry a marker which shows up well on the images of the imaging device, for example by virtue of an X-ray-opaque design of the active locator. Finally, the apparatus may also comprise a device for injecting a contrast agent in order that an emphasized depiction of the course of the vessels can be shown on the image.

In principle, the locating device used may be any of the systems known for this purpose. In particular, the locating device may determine the position and/or orientation of the associated locator by means of a mechanical, electromagnetic, optical and/or acoustic method.

According to an exemplary embodiment in a mechanical method, a change in the position of the locator is recorded by a mechanical connection thereto-e. g. via the catheter. Another mechanical method is based on the mass inertia, that is to say observing accelerations of the locator. By integration of this acceleration, it is possible to deduce the current position of the locator.

In electromagnetic location, use is made of spatially and/or temporally inhomogeneous electromagnetic fields. Preferably, in this case the locator may be a magnetic field sensor and the locating device may be a field generator for generating an inhomogeneous magnetic field, wherein the magnetic field sensor measures the magnitude and direction of the generated field and makes it possible to deduce from this its position relative to the field generator. However, the locator may also be the source of an (electro) magnetic field which is measured by at least one external sensor. Furthermore, electromagnetic locating devices may also measure the magnitude and direction of the ground magnetic field, in order to make it possible to deduce the position therefrom.

In optical methods, for example, a light source is observed from various locations so that the position of the light source can be deduced using stereoscopic methods.

In the present case, the near infrared (NIR) would be particularly suitable as light signal since it can to a certain extent penetrate body tissue.

Finally, acoustic methods are based on the transmission of sound waves, in particular ultrasound. For example, the active locator may be an ultrasound source, the signals of which are measured at least at three spatial points, wherein differences in propagation time make it possible to deduce the position of the locator. On the other hand, however, the locator may also be a receiver which measures the propagation times of sound signals from at least three spatially distributed sources and deduces its own position therefrom.

The plugging material which is injected into an aneurysm by means of the apparatus may in particular comprise an initially fluid, curable polymer material, plastic beads, a plastic coil, a hydrogel and/or a fibrin sponge. Numerous suitable materials are also described in US 6 024 754.

According to an exemplary embodiment a method of operting an apparatus for controlling the supply of a plugging material to a catheter during the therapeutic embolization of an aneurysm, which comprises the following steps:
a) Determining the position and/or orientation of the catheter via an active locator fitted thereon.
b) Automatically stopping the supply of the plugging material to the catheter if emergence of the catheter from the aneurysm is detected on account of the measurement data determined in step a).

The method implements in a general manner the steps which can be carried out using an apparatus of the type described above. Therefore, reference should be made to the above description with regard to details, advantages and further embodiments of the method. According to one further embodiment of the method, the position of the locator is recorded using a road map generated beforehand, in order in this way to check whether the catheter is correctly located in the aneurysm at the start of embolization.

Furthermore, the catheter and the aneurysm may optionally be imaged together at the start of embolization, for example by means of an X-ray image (rotation angiography or computer tomography) with administration of a contrast agent. The correct positioning of the catheter at the start of embolization can then be checked on the image.

According to one further embodiment of the method, the navigation of the catheter outside the aneurysm is assisted by determining the position and/or orientation of the active locator. In this way, the locator which is present in any case can additionally be used for further functions, and for example can help to reduce the amount of X-ray radiation to which the patient is exposed. A suitable method for navigation with the aid of active locators is described for example in US 5 042 486.

The invention will be further described with reference to examples of embodiments shown in the drawings to which, however, the invention is not restricted.
Fig. 1 schematically shows an apparatus according to the invention during the therapeutic embolization of an aneurysm.
Fig. 2 shows the diagram of Fig. 1 in the event of a complication.

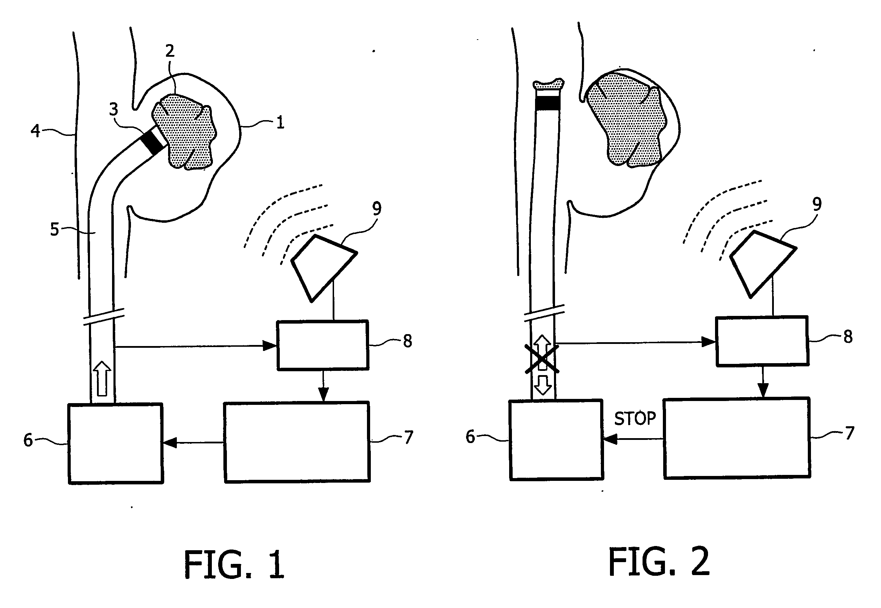

As can be seen in the schematic diagram of Fig. 1, during the therapeutic embolization of an aneurysm 1 in the wall of a vessel 4, a catheter 5 is inserted into the aneurysm. A suitable plugging material 2, such as a curable polymer for example, can then be injected into the aneurysm 1 via the catheter 5, in order to plug said aneurysm and end its negative effects on the blood flow. The plugging material 2 is supplied to the catheter 5 by means of a controllable pump device 6.

During injection of the plugging material 2, the problem arises that the catheter 5 tends to stretch and therefore to emerge from the aneurysm 1. If this happens, plugging material 2 is injected into the bloodstream and this entails the risk of blocking a vessel, damaging a vessel or causing life-threatening embolisms (apoplexy). When carrying out an embolization, great care is therefore taken to ensure that the catheter 5 remains in the aneurysm 1.

With the apparatus shown in the figure, in this connection a means is provided which facilitates monitoring of the catheter tip during the injection of plugging material and ensures safety even if, despite the greatest of care being taken, the catheter emerges from the aneurysm 1. For this purpose, the apparatus has an active locator 3 at the catheter tip, which active locator cooperates with a locating device arranged outside the body. In the example shown, the active locator 3 is a sensor for a magnetic field, wherein the field is generated outside the body by a field generator 9 which is fitted at a known spatial point. The field generator 9 and the active locator 3 are connected to a control unit 8 which is able, on account of the magnetic field strength and direction measured by the locator 3, to determine the current spatial position (coordinates) of the active locator 3.

Furthermore, the apparatus contains a monitoring unit 7 which may be for example a microcomputer and which is connected to the control unit 8 and the pump device 6. Unlike what is shown, parts of the apparatus, such as the monitoring unit 7 and the control unit 8 for example, may also be implemented by the same hardware (but different software).

Navigation of the catheter 5 in the vascular system and positioning thereof in the aneurysm 1 typically takes place by recording the measured positions of the locator 3 using a two-dimensional or three-dimensional road map generated beforehand by an X-ray angiograph. Moreover, directly before the start of filling, the aneurysm 1 may also be imaged together with the catheter 5 with administration of a contrast agent, and the positions of the aneurysm and the catheter are placed in relation to one another (recorded) using image processing means. In this case, the catheter preferably comprises an X-ray-opaque marker. The imaging device, for example an X-ray device, is not shown in the figures.

The monitoring unit 7 receives from the control unit 8 the information regarding the current position and/or orientation of the locator 3. By comparing this position/orientation with the position of the aneurysm 1 known from the start of the filling operation or the desired orientation of the catheter tip, it is then possible for the monitoring unit 7 to detect whether the catheter 5 is emerging from the aneurysm 1. If this happens, the monitoring unit 7 immediately controls the pump device 6 so that the latter stops the further supply of plugging material to the catheter 5 (Fig. 2). This reliably prevents significant amounts of plugging material from passing into the bloodstream. Optionally, the pump device 6 may also be designed or controlled to carry out brief back-suction, so that plugging material which has already escaped or is at the catheter tip can be sucked back in (small arrow in Fig. 2).

The active locator 3 present in the described apparatus may advantageously also be used for other functions. For example, it can be used to produce an amplified image of the aneurysm or to automatically determine an image region of interest. It is furthermore possible to use the locator 3 for navigation of the catheter in the vascular system and to show this on a road map.

## Claims

1. An apparatus for the therapeutic embolization of aneurysms (1), comprising:
- a catheter (5) for injecting a filling material (2) into an aneurysm (1);
- a locating device (8,9) and at least one active locator (3) fitted on the catheter (5), it being possible for the spatial position and/or orientation of the locator to be determined by the locating device (8,9) ;
- a pump device (6) for controllably supplying filling material (2) to the catheter (5);
- a monitoring unit (7) connected to the locating device (8) and the pump device (6), which monitoring unit is designed to detect emergence of the catheter from the aneurysm during the injection of plugging material (2) into the aneurysm (1), and thereupon to stop the supply of plugging material.

2. An apparatus as claimed in Claim 1, **characterized in that** the monitoring unit (7) contains a memory with a road map stored therein, and **in that** it is designed to record the measured position of the locator (3) using the road map.

3. An apparatus as claimed in Claim 1, **characterized in that** it comprises an imaging device such as in particular an X-ray device.

4. An apparatus as claimed in Claim 1, **characterized in that** the locating device (8, 9) is designed to determine the position and/or orientation of the active locator (3) by means of a mechanical, electromagnetic, optical and/or acoustic method.

5. An apparatus as claimed in Claim 4, **characterized in that** the active locator is a magnetic field sensor (3) and the locating device contains a field generator (9) for generating an electromagnetic field which is spatiallyand/or temporally inhomogeneous.

6. An apparatus as claimed in Claim 1, **characterized in that** the plugging material (2) comprises a curable polymer material, plastic beads, a plastic coil, a hydrogel and/or a fibrin sponge.

7. A method for operating an apparatus for controlling the supply of a plugging material (2) to a catheter (5) during the therapeutic embolization of an aneurysm (1) the method comprises:
a) determining the positionand/or orientation of the catheter via an active locator (3) fitted thereon; and
b) automatically stopping the supply of the plugging material (2) to the catheter (5) if emergence of the catheter from the aneurysm (1) is detected.

8. A method as claimed in Claim 7, **characterized in that** the position of the locator (3) is recorded using a road map generated beforehand.

9. A method as claimed in Claim 7, **characterized in that** the catheter (5) and the aneurysm (1) are imaged together at the start of embolization, preferably by means of X-rays or with administration of a contrast agent.

10. A method as claimed in Claim 7, **characterized in that** the navigation of the catheter (5) in the vascular system outside the aneurysm (1) is assisted by determining the position of the active locator (3).

## Patentansprüche

1. Gerät für die therapeutische Embolisation von Aneurysmen (1), das Folgendes umfasst:
- ein Katheter (5) zum Injizieren eines Füllmaterials (2) in ein Aneursyma (1);
- eine Lokalisierungsvorrichtung (8, 9) und mindestens einen an dem Katheter (5) angebrachten aktiven Positionsanzeiger (3), wobei es möglich ist, dass die räumliche Position und/oder Ausrichtung des Positionsanzeigers durch die Lokalisierungsvorrichtung (8, 9) ermittelt wird;
- eine Pumpenvorrichtung (6) zum steuerbaren Zuführen von Füllmaterial (2) zum Katheter (5);
- eine mit der Lokalisierungsvorrichtung (8) und der Pumpenvorrichtung (6) verbundene Überwachungseinheit (7), die vorgesehen ist, um das Austreten des Katheters aus dem Aneurysma während des Injizierens des Stopfmaterials (2) in das Aneurysma (1) zu detektieren und daraufhin die Zuführung von Stopfmaterial zu beenden.

2. Gerät nach Anspruch 1, **dadurch gekennzeichnet, dass** die Überwachungseinheit (7) einen Speicher mit einer darin gespeicherten Straßenkarte umfasst, und dass sie vorgesehen ist, um die gemessene Position des Positionsanzeigers (3) anhand der Straßenkarte aufzuzeichnen.

3. Gerät nach Anspruch 1, **dadurch gekennzeichnet, dass** es eine Bildgebungsvorrichtung, wie zum Beispiel insbesondere ein Röntgengerät, umfasst.

4. Gerät nach Anspruch 1, **dadurch gekennzeichnet, dass** die Lokalisierungsvorrichtung (8, 9) vorgesehen ist, um die Position und/oder Ausrichtung des aktiven Positionsanzeigers (3) mit Hilfe eines mechanischen, elektromagnetischen, optischen und/oder akustischen Verfahrens zu ermitteln.

5. Gerät nach Anspruch 4, **dadurch gekennzeichnet, dass** der aktive Positionsanzeiger ein Magnetfeldsensor (3) ist und die Lokalisierungsvorrichtung einen Feldgenerator (9) zum Erzeugen eines elektromagnetischen Feldes umfasst, das räumlich und/oder zeitlich inhomogen ist.

6. Gerät nach Anspruch 1, **dadurch gekennzeichnet, dass** das Stopfmaterial (2) ein aushärtbares Polymermaterial, Kunststoffkügelchen, eine Kunststoffspirale, ein Hydrogel und/oder einen Fibrinschwamm umfasst.

7. Verfahren zum Bedienen eines Geräts zum Steuern der Zuführung eines Stopfmaterials (2) zu einem Katheter (5) während der therapeutischen Embolisation eines Aneurysmas (1), wobei das Verfahren Folgendes umfasst:
a) Ermitteln der Position und/oder Ausrichtung des Katheters über einen daran angebrachten aktiven Positionsanzeiger (3); und
b) automatisches Beenden der Zuführung des Stopfmaterials (2) zum Katheter (5), wenn das Austreten des Katheters aus dem Aneurysma (1) detektiert wird.

8. Verfahren nach Anspruch 7, **dadurch gekennzeichnet, dass** die Position des Positionsanzeigers (3) anhand einer zuvor erstellten Straßenkarte aufgezeichnet wird.

9. Verfahren nach Anspruch 7, **dadurch gekennzeichnet, dass** der Katheter (5) und das Aneurysma (1) zusammen zu Beginn der Embolisation abgebildet werden, vorzugsweise mittels Röntgenstrahlen oder mittels Verabreichung eines Kontrastmittels.

10. Verfahren nach Anspruch 7, **dadurch gekennzeichnet, dass** die Navigation des Katheters (5) im Gefäßsystem außerhalb des Aneurysmas (1) durch Ermitteln der Position des aktiven Positionsanzeigers (3) unterstützt wird.

## Revendications

1. Dispositif pour l'embolisation thérapeutique d'anévrismes (1), comprenant :
- un cathéter (5) pour injecter un matériau de remplissage (2) dans un anévrisme (1) ;
- un dispositif de localisation (8, 9) et au moins une balise de localisation active (3) montée sur le cathéter (5), la position dans l'espace et/ou l'orientation de la balise de localisation pouvant être déterminée par le dispositif de localisation (8, 9) ;
- un dispositif de pompe (6) pour fournir un matériau de remplissage (2) au cathéter (5) de façon contrôlée ;
- une unité de surveillance (7) reliée au dispositif de localisation (8) et au dispositif de pompe (6), ladite unité de surveillance étant conçue pour détecter que le cathéter est sorti de l'anévrisme pendant l'injection du matériau de colmatage (2) dans l'anévrisme (1), et pour arrêter dès cet instant la fourniture du matériau de colmatage.

2. Dispositif selon la revendication 1, **caractérisé en ce que** l'unité de surveillance (7) contient une mémoire dans laquelle est stockée un plan, et **en ce qu'**elle est conçue pour enregistrer la position mesurée de la balise de localisation (3) au moyen du plan.

3. Dispositif selon la revendication 1, **caractérisé en ce qu'**il comprend un dispositif d'imagerie tel, en particulier, qu'un dispositif de radiographie.

4. Dispositif selon la revendication 1, **caractérisé en ce que** le dispositif de localisation (8, 9) est conçu pour déterminer la position et/ou l'orientation de la balise de localisation active (3) au moyen d'un procédé mécanique, électromagnétiques, optique et/ou acoustique.

5. Dispositif selon la revendication 4, **caractérisé en ce que** la balise de localisation active est un capteur de champ magnétique (3) et le dispositif de localisation contient un générateur de champ (9) destiné à produire un champ électromagnétique qui est hétérogène dans l'espace et/ou dans le temps.

6. Dispositif selon la revendication 1, **caractérisé en ce que** le matériau de colmatage (2) comprend un matériau polymère durcissable, des billes de plastique, une bobine de plastique, un hydrogel et/ou une éponge en fibrine.

7. Procédé d'utilisation d'un dispositif destiné à contrôler la fourniture d'un matériau de colmatage (2) à un cathéter (5) pendant l'embolisation thérapeutique d'un anévrisme (1), le procédé comprenant les étapes consistant à :
a) déterminer la position et/ou l'orientation du cathéter par l'intermédiaire d'une balise de localisation active (3) montée sur celui-ci ; et
b) arrêter automatiquement la fourniture du matériau de colmatage (2) au cathéter (5) s'il est détecté que le cathéter est sorti de l'anévrisme (1).

8. Procédé selon la revendication 7, **caractérisé en ce que** la position de la balise de localisation (3) est enregistrée au moyen d'un plan produit préalablement.

9. Procédé selon la revendication 7, **caractérisé en ce qu'**une image à la fois du cathéter (5) et de l'anévrisme (1) est formée au début de l'embolisation, de préférence au moyen de rayons X ou par administration d'un agent de contraste.

10. Procédé selon la revendication 7, **caractérisé en ce que** la navigation du cathéter (5) dans le système vasculaire à l'extérieur de l'anévrisme (1) est aidée par la détermination de la position de la balise de localisation active (3).
